# EUROPEAN PATENT SPECIFICATION

(11) **EP 2 870 252 B1**
(45) Date of publication and mention of the grant of the patent: **22.01.2020**
(21) Application number: 13739848.3
(22) Date of filing: 03.07.2013
(51) Int. Cl.: C12P 7/64, A23C 19/04, A23C 19/05, A23C 19/06

(54) **METHODS USING PATATIN**
METHODEN UNTER VERWENDUNG VON PATATIN
MÉTHODES UTILISANT LA PATATINE

(30) Priority: 04.07.2012 EP 12174894
(43) Date of publication of application: 13.05.2015
(73) Proprietor: Coöperatie AVEBE U.A., 9641 GK Veendam (NL)
(72) Inventor: SPELBRINK, Robin Eric Jacobus, NL-9717 JR Groningen (NL); GIUSEPPIN, Marco Luigi Federico, NL-9461 JB Gieten (NL); EGMOND, Maarten Robert, NL-3511 GW Utrecht (NL)
(74) Representative: V.O.
(86) International application number: PCT/NL2013/050488
(87) International publication number: WO 2014/007621

(56) References cited:
- US-A1- 2009 053 191
- US-A1- 2011 311 679
- COREY ANDERSON ET AL: "Hydrolytic selectivity of patatin (lipid acyl hydrolase) from potato (Solanum tuberosum L.) tubers toward various lipids", JOURNAL OF FOOD BIOCHEMISTRY, vol. 26, no. 1, 1 February 2002 (2002-02-01), pages 63-74, XP055044643, ISSN: 0145-8884, DOI: 10.1111/j.1745-4514.2002.tb00050.x
- HOANG J. H. B. HIRSCHBERG ET AL: "Cloning, expression, purification and characterization of patatin, a novel phospholipase A", EUROPEAN JOURNAL OF BIOCHEMISTRY, vol. 268, no. 19, 1 October 2001 (2001-10-01), pages 5037-5044, XP055044636, ISSN: 0014-2956, DOI: 10.1046/j.0014-2956.2001.02411.x cited in the application
- PRAPHAN PINSIRODOM ET AL: "Fatty acid and product selectivities of potato tuber lipid acyl hydrolase in esterification reactions with glycerol in organic media", JOURNAL OF THE AMERICAN OIL CHEMISTS' SOCIETY, vol. 76, no. 10, 1 October 1999 (1999-10-01), pages 1119-1125, XP055044701, ISSN: 0003-021X, DOI: 10.1007/s11746-999-0083-6
- KLEIN ROBERT R ET AL: "Altered acyl chain length specificity of Rhizopus delemar lipase through mutagenesis and molecular modeling", LIPIDS, SPRINGER, DE, vol. 32, no. 2, 1 January 1997 (1997-01-01), pages 123-130, XP009088258, ISSN: 0024-4201, DOI: 10.1007/S11745-997-0016-1
- NELSON ET AL: "Pregastric esterase and other oral lipases", JOURNAL OF DAIRY SCIENCE, vol. 60, no. 3, March 1977 (1977-03), pages 327-362, XP002953468,
- VAN KONINGSVELD GERRIT A ET AL: "Effects of protein composition and enzymatic activity on formation and properties of potato protein stabilized emulsions", JOURNAL OF AGRICULTURAL AND FOOD CHEMISTRY,, vol. 54, no. 17, 1 August 2006 (2006-08-01), pages 6419-6427, XP002553907, DOI: 10.1021/JF061278Z

## Description

### Introduction

In the production of food products, the breakdown of lipids and other esters is important for flavour, structure, texture or purity. This process, called hydrolysis, results in free fatty acids, that among others are important contributors to flavour, *i*.*e*. the combination of taste and smell. In particular short and medium chain fatty acids, which occur in relatively large quantities in milk fat, contribute strongly to the flavour of food.

While esters can be hydrolysed chemically using either strong bases or acids, such procedures are crude and non-specific, resulting in loss of yield, undesired side products and waste. Enzymatic hydrolysis is generally more specific, avoiding at least part of these problems. Lipases are surface enzymes that are often selective for the hydrolysis of specific lipids in terms of fatty acid chain length or fatty acid chain position. This specificity determines how appropriate a specific lipase is for any given application.

The use of lipases is common in industry. Examples include racemic drug resolution, fat and lipid modification, flavour synthesis and the production of pharma- and nutraceuticals. The vast majority of lipases that are presently in use are obtained using fungal or bacterial fermentation systems, although animal-derived lipases see some use as well. Plant-derived lipases are relatively rare for industrial applications.

Most industrially applicable lipases are obtained by bacterial fermentation and require cumbersome downstream processing. Such lipases tend to be of low purity on both a total- and protein-basis and therefore contain high amounts of carbohydrates, salts and possible undesired side-activities. Furthermore, commercial lipase preparations generally contain a broad variety of non-protein material, salts and non-lipase enzymes (Bjurlin et al 2001 JAOCS 78-2 p 153-160).

The use of lipases to produce flavour in a food product such as cheese is quite common, and many different lipase preparations have been reported. Traditionally, the digestive enzymes from calves or pigs, called rennet, would be added to cheese, because rennet is able to induce coagulation of milk, and because rennet induces the production of free fatty acids, which confer flavour to cheese. This process involves expensive enzyme isolation requiring dead calves or pigs, which is cumbersome and unsuitable for vegetarians. Also, it introduces the possibility of transfer of diseases.

Generally, cheese is made by coagulation of milk, for instance by the addition of rennet and/or acid. Upon coagulation, milk separates into curd and whey. Whey, a watery solution of milk proteins, is discarded, while the curd is collected and gently pressed to remove some of the remaining water. Still, the curd obtained contains approximately 30 % water, and should be considered a colloidal dispersion of milk fat, proteins and water. Suitable enzymes are added, either to the milk or to the curd (e.g. in the form of rennet). For most cheeses, the curd is subsequently allowed to ripen.

In the making of cheese, the action of enzymes starts the primary degradation pathway of milk constituents by among others glycolysis, proteolysis and lipolysis. This leads to a range of compounds that are responsible for cheese flavour, such as for instance amino acids, certain peptides, esters, aldehydes, ketones, phenols and fatty acids. In the group of fatty acids, especially the short- and medium chain fatty acids are responsible for positive flavour development, while the longer chain fatty acids lead to a soapy, unpleasant taste. Different fatty acids convey a different type of taste to a cheese. For example, C₄ fatty acids have high presence in cow milk's cheese, whereas C₆ fatty acids are common in goat cheese. However, the presently used lipases display little or no selectivity for releasing the desired flavour components, and avoiding the undesired soapy flavours.

At present, microbial lipases are preferred for cheese-making, and in general use is made of genetically modified bacteria to obtain one specific type of lipase. Such lipases require complicated and expensive isolation methods and may lead to rancidity in the final product because the reaction often has to be continued for too long time.

A plant-derived lipase enzyme can be obtained from potato. Potato proteins can be divided into three categories: (i) the patatin family, highly homologous acidic 43 kDa glycoproteins (the high-molecular weight fraction, "HMW", making up 40-50 wt.% of the potato proteins), (ii) basic 5-25 kDa protease inhibitors ("PI", 30-40 wt.% of the potato proteins) and (iii) other proteins mostly high molecular weight proteins (10-20 wt.% of the potato proteins). The patatin family is known to have some lipase activity, and can be obtained via a single-step chromatographic process followed by concentration and drying. A highly convenient process for the isolation of among others patatin with high purity is described in application WO2008/069650.

In practice, potato proteins, including lipase, have been mainly used as feedstock for animals because of a lack of practical commercial use. Generally, practical use of patatin has been considered limited because patatin is inactive towards triglycerides (see for instance Hirschberg et al, Eur. J. Biochem 2001, 268, 5037, Galliard et al., Biochem. J. 1971, 121, 379 or Andrews et al, Biochem J. 1988, 252, 199), even though it has esterase activity on among others phospholipids and monoglycerides. The document Van Koningsveld et al. (2006), Journal of Agricultural and Food Chemistry, vol. 54, no. 17, p. 6419-6427 discloses the hydrolysis of emulsified tricaprylin by patatin.

### Summary of the invention

The invention provides a means of using patatin in an aqueous phase for the hydrolysis of specific types of lipids. It has been found that patatin is capable of hydrolysing a C₄ - C₈ fatty acid from a triglyceride. However, it will not, or essentially not, hydrolyse glycerol fatty acid esters of longer chain lengths. Thus, patatin displays a surprising selectivity for short- and medium chain fatty acids, especially in aqueous environment. It has been found that this selectivity allows for high practical value in the making of cheese.

Accordingly, the present invention relates to:
A method for hydrolysing a fatty acid from a triglyceride that contains at least one C4-C8 fatty acid in an emulsion or colloidal dispersion comprising milk fat, comprising subjecting the triglyceride to patatin derived from potato *(Solanum tuberosum)* in the presence of water, wherein the patatin hydrolyses a C4 - C8 fatty acid.

In a second embodiment, the invention relates to:
A method of making cheese, comprising the steps of
- coagulating milk to obtain a curd,
- draining the curd, and
- forming a fresh cheese, optionally followed by a ripening step,
wherein patatin derived from potato *(Solanum tuberosum)* is added to the milk or to the curd to hydrolyse a C4 - C8 fatty acid according to the method described above.

### Brief description of the figures

Figure 1: Percentage of test panel that reports difference between patatin-treated cheese and a reference cheese at the indicated patatin concentration in the milk before coagulation.
Figure 2: Distribution of patatin over curd and whey via a labelling approach. A: Milk prior to coagulation. B: Milk after coagulation. C: Milk containing 2 g/L unlabelled patatin before coagulation. D: Milk containing 2 g/L unlabelled patatin after coagulation.. E: Milk containing 2 g/L Coomassie R-250-labelled patatin before coagulation. F: Milk containing 2 g/L Coomassie R-250-labelled patatin after coagulation.. G: Milk containing 2 g/L Coomassie G-250-labelled patatin before coagulation. H: Milk containing 2 g/L Coomassie G-250-labelled patatin after coagulation.
Figure 3: Lipase activity recovered in whey after rennet-coagulation at different patatin doses.
Figure 4: Dose-response relationship between patatin dose and sensory attribute.
Figure 5: Preference of patatin towards the hydrolyis of fatty acid from cheese fat.
Figure 6: Increase in FFA derived volatile cheese favour compounds after 6 weeks (6a) and after 13 weeks (6b).
Figure 7: Lipase activity in HMW potato protein powder over two years.

### Detailed description

For the scope of the present invention, patatin is understood to mean the high molecular weight (HMW) fraction of native potato protein isolates, a highly homologous family of glycoproteins having a molecular weight of 30 kDa or more, preferably 35 kDa or more and more preferably of about 43 kDa, and an isoelectric point of less than 5.8, preferably between 4.8 and 5.5, which makes up about 40-50 wt.% of the potato proteins. Patatin is a family of glycoproteins that displays acyl-hydrolase reactivity and accounts for up to 40 wt % of the total soluble protein in potato tubers. In application WO 2008/069650 an elaborate description of the isolation of patatin from potato fruit juice (PFJ) or potato fruit water (PFW) is described, which is included herein by reference.

The process of WO 2008/069650 entails subjecting potato fruit juice to a flocculation by a divalent metal cation at a pH of 7-9, and centrifuging the flocculated potato fruit juice, thereby forming a supernatant. Subsequently, the supernatant is subjected to expanded bed adsorption chromatography operated at a pH of less than 11, and a temperature of 5-35 °C using an adsorbent capable of binding potato protein, thereby adsorbing the native potato protein to the adsorbent. Finally, at least one native potato protein isolate is eluted from the adsorbent with an eluent. This method results among others in isolated native patatin of high purity, with a minimum of denatured protein present and characterised by a stable solubility.

The potato fruit juice is pre-treated with a divalent metal cation at a pH of 7-9, preferably 7.0-7.5, to flocculate undesired material, followed by a separation of the flocks by centrifugation. A particularly suitable divalent metal cation is Ca²⁺. This pre-treatment removes undesired material such as negatively charged polymers, pectins, glycoalkaloids, and micro-organisms from the potato fruit juice. In particular, the removal of pectins and glycoalkaloids is advantageous, since these compounds adhere to the potato proteins and may cause flocculation, thereby leading to an unstable protein isolate in terms of solubility and other physical properties.

In the second step of the process, the supernatant is subjected to expanded bed adsorption chromatography. It is advantageous to keep the temperature of the starting material below 35 C for a better stability of patatin. Furthermore it is preferred to use a moderately high flow rate, typically in the range of 600-1200 cm/h. The expanded bed adsorption chromatography is operated at a pH of less than 11, preferably at a pH of less than 10.

The native potato proteins in the pre-treated potato fruit juice are isolated from the supernatant by binding them onto a suitable adsorbent in the expanded bed adsorption column. Column materials that bind certain amounts of native potato proteins include mixed-mode adsorbentia such as Amersham Streamline™ Direct CST I (GE Healthcare), Fastline adsorbentia (Upfront Chromatography A/S), macroporous adsorbentia such as Amberlite™ XAD7HP (Röhm & Haas Company) and ion exchange adsorbents. The adsorbent with adsorbed native potato proteins is subsequently eluted with a suitable eluent in order to retrieve the native potato protein isolate, such as patatin. The eluent preferably has a pH in the range of 4-12, more preferably in the range of 5.5-11.0.

In a preferred embodiment using mixed-mode adsorbentia the proteins can be fractionated to both isoelectric point and molecular weight. This allows separation of for instance patatin and protease inhibitor fractions. Patatin isolates are eluted at a pH of 5.7-8.7, preferably at a pH of 5.8-6.5.

Acyl-hydrolase reactivity is generally understood as the ability of a (class of) enzyme(s) to catalyse the hydrolysis of an ester bond by a water molecule to form the constituent carboxylic acid and alcohol. This reaction can sometimes be reversed by suitable adaptation of the reaction conditions, in which case esterification of a carboxylic acid and an alcohol occurs. Reaction conditions that may influence the direction of the reaction include for instance temperature, reactant identity, and/or the amount of water, carboxylic acid and alcohol present. The present invention discloses that patatin has highly selective acyl-hydrolase reactivity, which makes it highly suitable for cheese-making.

The hydrolase activity of patatin is directed specifically at acyl groups as found in lipids, in particular mono-, di and triacylglycerides. The hydrolase activity of patatin was known to be strong for monoglycerides. It was, however, also reported that no such activity was found for triglycerides (see for instance Hirschberg et al, Eur. J. Biochem 2001, 268, 5037, Galliard et al., Biochem. J. 1971, 121, 379 or Andrews et al, Biochem J. 1988, 252, 199). In accordance with the present invention it has surprisingly been found that, despite these reports, there actually is triglyceride hydrolase activity for patatin, and that this activity is very specific for C₄ - C₈ fatty acids.

A fatty acid is a class of compounds, characterised by the presence of a 1-positioned carboxylic acid group on a further linear carbon chain. The length of the carbon chain is an important characteristic of a fatty acid, so that a fatty acid having a carbon chain of 10 consecutive linear carbon atoms is called a C₁₀ fatty acid. Generally, the fatty acids known are C₄ - C₃₆ fatty acids. The carbon chain can be saturated but may also comprise one or more double bonds.

Fatty acids are traditionally divided into several groups, using different methods. One method is to divide them according to their degree of saturation. Then, one group of fatty acids is defined as saturated fatty acids, which do not have double bonds between any two carbon atoms in their carbon chain.

Unsaturated fatty acids have one or more double bonds in the carbon chain. Within the unsaturated fatty acids, there exist mono-unsaturated fatty acids (MUFA), having one double bond in the carbon chain, and polyunsaturated fatty acids (PUFA), having multiple double bonds in the carbon chain.

Fatty acids may also be divided according to the length of their carbon chain. Thus, there exist short chain fatty acids (SCFA), which have a carbon chain of less than 6 carbon atoms. Medium-chain fatty acids (MCFA) have a carbon chain of 6 - 12 carbon atoms, and long chain fatty acids (LCFA) have a carbon chain of 13-21 carbon atoms. Very long chain fatty acids (VLCFA) have a carbon chain of longer than 22 carbon atoms. When classifying fatty acids according to chain length, the carbon chain may be saturated, or mono- or poly unsaturated.

Fatty acids are present inside all living organisms, and have several functions. Usually, these functions are exerted by one or more fatty acids incorporated into a larger molecule. Thus, fatty acids can be connected to sugars, amino acids or glycerol-derivatives, and have functions ranging from energy storage to cell structuring, and many more.

A lipid, for the scope of this invention, is any compound in which a fatty acid is linked through an ester bond to a hydroxyl group of glycerol. Monoacyl-glycerides (MAG or monoglyceride) are esters of glycerol with one fatty acid and two free hydroxyl groups. Diacylglycerides (DAG or diglyceride) are esters of glycerol with two fatty acids and one free hydroxyl group. Triacylglycerides (TAG or triglyceride) are esters of glycerol with three fatty acids. Triacylglycerides are colloquially referred to as "fat"; oil is also a fat but "fat" is generally used to refer to solid or semi-solid triglycerides, whereas "oil" is used for liquid or viscous triglycerides.

Many different fatty acids exist, and the relative abundance of the various fatty acids is more or less constant within the same species.The distribution of the various fatty acids over the triglycerides is more or less random. Thus, a triglyceride generally comprises three different fatty acids, but the statistical chance of finding a triglyceride having two identical fatty acids is non-negligible. Furthermore, triglycerides with three identical fatty acid units occur naturally. An example is tributyrin (three C₄ fatty acids on a single glycerol backbone), which is known to exist in butter.

All the esters of glycerol and one or more fatty acids as discussed above can be referred to as "lipid". However, lipids also include MAGs and DAGs in which a free hydroxyl group of the MAG- or DAG-glycerol is coupled to another group, such as a phosphate group. A molecule in which two fatty acids and a phosphate group are coupled to a single glycerol molecule is referred to as a phospholipid, whereas a molecule in which only one fatty acid is coupled to glycerol, leaving a free hydroxyl-group, is referred to as a lysophospholipid. A phospholipid or a lysophospholipid can have further substitution on the phosphate group. For example, a phospholipid or a lysophospholipid whose phosphate group is further functionalised with choline is referred to as a phosphatidylcholine, and this therefore is a type of phospholipid, and also a type of lipid.

The term lipid colloquially further includes compounds in which one or more fatty acids are coupled through an ester bond to a sugar, such as a monomeric, dimeric or polymeric sugar. In this case, it is called a fatty acid carbohydrate ester or glycolipid. Also, lipids may comprise other esters of fatty acids, such as sterol esters. However, for the scope of the present invention the term lipid is limited to fatty acid esters of glycerol; other esters of fatty acids such a with sugars or sterols are considered not part of the group of lipids.

Lipids are generally separated into two groups: polar lipids, and neutral lipids. The polar lipids dissolve in water to form, for instance, micelles or bilayers, whereas the neutral lipids display very low water solubility. Phospholipids are considered polar lipids, and generally have an octanol-water coefficient (LogP) between 5 and 10. Neutral lipids have low water solubility, and in general have a LogP that is higher than 10. Triglycerides having a lower octanol-water coefficient do exist however, such as for instance triacetin (LogP = 0.25), tributyrin (C₄, LogP = 3.27), tricaprion (C₆, LogP = 5.6), tricaprylin (C₈, LogP = 9.2); tricaprin (C₁₀) has LogP = 10.9. It has been found that patatin is effective in hydrolyzing relatively polar triglycerides, *i*.*e*. those with LogP lower than 10 (*vide infra*).

For the scope of the present invention, the term triglycerides includes monoglycerides, diglycerides and triglycerides. Because monoglycerides and diglycerides do essentially not occur naturally, but form by hydrolysis of tryglycerides, the breakdown of neutral lipids always starts with the breakdown of triglycerides. Thus, in a process of hydrolysing neutral lipids, it is advantageous when hydrolysis of triglycerides occurs.

There are several types of mixtures. Mixture types important in the context of the present invention are:
- solutions
- colloidal dispersions
- emulsions
- suspensions

The Tyndall-effect is the elastic scattering of light by particles that are bigger than the wavelength of the light used. It is mostly known from colloidal dispersions and suspensions, and can be used to determine the particle size in such media as is known in the art.

A system, as used herein, can be any liquid or semi-liquid environment that will allow for chemical reactions to occur by diffusion of the reactants so that they can "find each other" and interact It can take the shape of a homogeneous solution, a suspension, a water-in-oil or an oil-in-water emulsion, and a colloidal dispersion, either in liquid or in highly viscous, apparent solid form.

Solutions are mixtures made by mixing a solute and a solvent. The solute is the substance that dissolves. The solvent is the substance that does the dissolving. Solutions are homogeneous and do not show the Tyndall effect.

Colloidal dispersions are mixtures in which two or more immiscible phases are present, so that one phase (a distributed or internal phase) is distributed within another (the continuous phase). Additional immiscible phases may also be present. The internal phase may be liquid, solid or gas, and similarly the continuous phase may be liquid, solid or gas, with the exception that gas-gas dispersions do not exist. Colloidal dispersions appear homogeneous but are actually heterogeneous. However, the one or more internal phases are distributed homogeneously in the continuous phase(s). A characteristic for the scope of this invention is that colloidal dispersions do not settle when left standing undisturbed, as long as no chemical changes in composition occur. Curd and milk are examples of colloidal dispersions. Colloidal dispersions do show the Tyndall effect.

Emulsions are heterogeneous mixtures of at least two immiscible liquids. Because a liquid distributed phase is distributed in a liquid continuous phase, emulsions share this feature with colloidal dispersions. For the scope of this invention however, emulsions will settle into their constituent phases when they are left standing undisturbed for long enough time. Thus, an emulsion is distinguished from a colloidal dispersion by the time it remains a stable system in which one phase is distributed homogeneously in another.

Often, some form of stabilizer is added to an emulsion, which precludes separation into layers. However, if without such stabilization the system would in time separate into two layers, such systems are considered an emulsion, at least until separation occurs. Emulsions show the Tyndall effect.

Suspensions are heterogeneous mixtures of a solid and a liquid in which the solid does not dissolve. Hence they comprise at least two phases in which one phase is distributed homogenously in another, and share this characteristic with a colloidal dispersion. Suspensions, in the terminology of this invention, have larger particles than a solid-liquid colloidal dispersion, and will settle when left standing undisturbed. Suspensions show the Tyndall effect.

This invention discloses a method for hydrolysing a fatty acid from a triglyceride that contains at least one C₄ - C₈ fatty acid in an emulsion or colloidal dispersion comprising milk fat, comprising subjecting the triglyceride to patatin derived from potato (*Solanum tuberosum*) in the presence of water, wherein the patatin hydrolyses a C₄ - C₈ fatty acid, or catalyses the hydrolysis of a C₄ - C₈ fatty acid.

In a triglyceride mixture comprising different fatty acids, hydrolysis occurs selectively. This is interpreted as that essentially only a C₄ - C₈ fatty acid is hydrolysed from the glycerol backbone. Thus, the patatin aids to break the ester bond between the C₄ - C₈ fatty acid and the glycerol backbone. Fatty acids with a shorter or longer carbon chain are essentially not hydrolysed and remain attached to the glycerol backbone in the presence of patatin. Preferably, C₄ and C₆ fatty acids are hydrolysed, and most preferred is hydrolysis of only C₄ fatty acids, especially in the making of cow's milk cheese, or of only C₆ fatty acids, especially in the making of goat cheese.

The position on the glycerol backbone in which the fatty acid is located is of no influence. The outer fatty acid positions (sn(1) and (sn(3)) are hydrolysed somewhat more efficient, but also the fatty acid located at the centre (sn(2)) position can be hydrolysed. Thus, preferably, the outer positions of the glycerol backbone are hydrolysed with preference over the centre position.

The degree of saturation of the fatty acid is not relevant, and both saturated and unsaturated C₄ - C₈ fatty acids can be hydrolysed using the present invention. However, unsaturated C₄ - C₈ fatty acids are rare, so that preferably, saturated C₄ - C₈ fatty acids are hydrolysed according to the present invention.

Hydrolysis according to the present invention thus results in free C₄ - C₈ fatty acids and diglycerides. Subsequently, hydrolysis may continue to form more free fatty acids and monoglycerides and glycerol. Hydrolysis of C₄ - C₈ fatty acids according to the invention may be stopped at any suitable time before hydrolysis is finished.

Selective hydrolysis of triglycerides may be limited to those triglycerides that have only C₄ - C₈ fatty acids on their glycerol backbone. Preferably in this case, the fatty acids at the outer positions on the glycerol backbone are selectively hydrolysed. Preferably, the fatty acids for hydrolysis are C₄ and C₆ fatty acids, and most preferred is hydrolysis of only C₄ fatty acids, especially in the making of cow's milk cheese, or of only C₆ fatty acids, especially in the making of goat cheese.

The water-solubility of the triglycerides to be hydrolysed according to the present invention is best described using the octanol-water partioning coefficient logP. LogP of triglycerides to be hydrolysed with a method according to the present invention is equal to or lower than 10, preferably lower than 9.2, preferably lower than 6.3. Even more preferably, logP should be in the range of 0.25 to 6.3 and even more preferably in the range of 3.27 to 6.3. LogP, for the scope of the present invention, is defined as the partitioning coefficient of the triglyceride between octanol and demineralised water at 25 °C, which can routinely be determined by those skilled in the art, for example by using the shake-flask method.

Natural fat or natural oil is mainly composed of triglycerides, with a wide range of different fatty acids of varying chain length. Natural fat or natural oil may however contain impurities, such as diglycerides, which form by hydrolysis of triglycerides. The various fatty acids present in natural fats and oils may have a carbon chain of 4 to 28 carbon atoms. The fatty acid profile of a particular type of fat may be determined using any method, such as for instance by a GC-based method according to de Jong and Badings, 1990, J High Resolution Chrom. 13:94-98. The present method is preferentially directed at the hydrolysis of natural fat or natural oil.

The patatin used in the present invention is derived from potato Solanum tuberosum. Preferably, the patatin is isolated from potato juice obtained after starch milling. The potato juice originates from all types of potato cultivars both for industrial starch production or direct human consumption or feed. Preferably, the patatin is isolated from the potato juice, and preferably it is also purified, such as from other potato proteins and impurities. Further it is preferred for a method according to the present invention to use patatin in native form, *i*.*e*. not denatured. Most preferably, native patatin freely dissolved or dispersed in an aqueous phase is used.

Hydrolysis of esters according to the present invention can be done in any solvent or without solvent, as long as sufficient water is present to allow the hydrolysis to proceed. It is an important aspect of the present invention that while most lipases function on the interface between hydrophilic and hydrophobic areas, patatin functions best in an aqueous phase. Therefore, the method of the present invention pertains to the hydrolysis of a triglyceride by patatin in an emulsion or colloidal dispersion, to allow sufficient contact between the apolar triglycerides in the hydrophobic phase, and the patatin in the water phase. Also, the aqueous phase may be the continuous phase in a multiphase system. Practical uses that have so far been identified for patatin, include an aqueous solution, suspension or emulsion, wherein it is not important whether this is a water-in-oil emulsion or an oil-in-water emulsion. Most preferably however, patatin is used in a method to hydrolyse one or more fatty acids from a triglyceride in a colloidal dispersion or an emulsion. In case an emulsion is used, this is preferably a water-in-oil emulsion.

Sufficient presence of water is a prerequisite for the invention to allow hydrolysis of a C₄ - C₈ fatty acid off the glycerol backbone of the triglyceride in any system. Sufficient water in this respect means a water content of at least 1 vol. %, preferably at least 5 vol.%, more preferably at least 10 vol.%,more preferably at least 15 vol.%, more preferably at least 20 vol.%, and most preferably at least 25 vol.%, calculated as a percentage of the full system.

Temperatures suitable for hydrolysing one or more C₄ - C₈ fatty acids are preferably temperatures at which patatin is active, such as 4-80 °C, preferably 10 - 65 °C. For processes where slow reaction rates give the best result, such as cheese making, it is better to use a temperature of 11-23 °C, preferably 13 °C. For a process where a higher reaction rate is desired it is best to use a temperature just below denaturation conditions, such as for instance 50 - 65°C.

Similarly, the pH should be so that patatin is active; suitable pH-values at which the hydrolysis of a C₄ - C₈ fatty acid proceeds are between 4,5 and 9, preferably 8.5. For cheese making, the optimum pH to use is between 4.8 and 6.7.

According to the invention, the emulsion or colloidal dispersion in which hydrolysis of a C₄ - C₈ fatty acid occurs comprises milk fat. Milk fat is naturally rich in short- and medium chain triglycerides, for which reason patatin is highly suitable for selective hydrolysis of milk. Therefore, the emulsion or colloidal dispersion preferably comprises milk.

Any type of milk that contains triglycerides comprising C₄ - C₈ fatty acids can be hydrolysed by patatin. Thus, any mammal's milk is appropriate, including cow, sheep, goat, donkey, horse, buffalo, yak, reindeer, camel and moose. Preferably however, cow, sheep or goat milk is used in combination with patatin, in particular cow milk.

In an alternative preferred embodiment, the emulsion or colloidal dispersion of the invention is curd. Curd is a colloidal dispersion obtained from milk, comprising triglycerides, proteins and approximately 30 % water. The triglycerides comprise a relatively high abundance of C₄ - C₈ fatty acids, which makes them highly suitable for selective hydrolysis by patatin. Curd takes the shape of a viscous solid-like mass. It is, amongst others, used for the making of cheese.

By adding patatin to an emulsion or colloidal dispersion used in a food production process, such as preferably milk or curd, to hydrolyse C₄ - C₈ fatty acids, the short- and medium chain fatty acid flavour components are released from the glycerol backbone at higher rate than with other lipases. Flavour in this respect is a combination of taste and smell. This allows for the use of patatin to enhance the flavour of a food product. In accordance with this embodiment, the hydrolysis is carried out in a system which is, or is part of, or is a starting material for the food product. Preferably, this food product is cheese, most preferably Italian-type cheese, blue cheese or enzyme-modified cheese. In this preferred embodiment, hydrolysis is for instance carried out in the milk used for making the cheese and thus form part of a method of preparing this food product, *i*.*e*. cheese.

Use of patatin in the cheese-making process has considerable advantages over the use of other lipases, such as microbial lipases or rennet. The specificity for C₄ - C₈ fatty acids results in an accelerated cheese production process, and/or in cheese having enhanced flavour. For this reason, curd or milk with added patatin can conveniently be used for cheese-making.

When patatin is used in a method for cheese-making, it can be added in any or all phases of cheese-making. It can be added to curd directly, but preferably patatin is added to milk before coagulation. It will fractionate mainly with the curd during coagulation. The curd obtained contains approximately 30 % water, and should be considered a colloidal dispersion of milk fat, proteins and water. The advantage of using patatin over the use of enzymes of other sources is that patatin has high selectivity for the release of C₄ - C₈ fatty acids from milk fat triglycerides, increasing the speed with which the cheese ripens, and increasing flavour development, thereby enhancing the flavour. At the same time, hydrolysis of longer chain-length fatty acids, that confer a soapy taste to cheese, is prevented because patatin has no reactivity towards such substrates. Also, patatin prevents runaway reactions that can cause rancidity due to overly extensive hydrolysis, because it hydrolyses flavour-releasing fatty acids selectively, without hydrolysing other fatty acid esters of glycerol. Finally, in contrast to many microbial lipases, patatin is easily deactivated.

Raising the temperature to common pasteurisation temperatures such as for instance between 50 and 80 °C, preferably 70-75 °C and more preferably 75 °C, essentially inactivates patatin. At 75 °C, 90 % reduction in activity is seen within at most 10 s, whereas at 70 °C, 90 % reduction in activity is seen within 17 s. A pH-dependency is observed, resulting in longer deactivation times with lower pH, but at pH of 6.7, the activity of patatin is reduced to 90 % within 8.2 s at 75 °C.

Patatin can be used in combination with other enzymes, such as for instance natural rennet or microbial rennet, thereby increasing the ripening speed of cheese and conferring increased flavour development. It can be used in combination with any type of milk coagulation. Therefore, enzymatically curdled cheese, which was coagulated either by rennet or by microbial enzymes, benefits from the addition of patatin. Also acid-curdled cheese, as well as whey cheese, benefits from added patatin because of its specific hydrolysis properties.

Accordingly, a method for the production of cheese comprising the use of patatin is disclosed in the present invention. This method comprises the steps of
- coagulating milk to obtain a curd,
- draining the curd, and
- forming a fresh cheese, optionally followed by a ripening step
wherein patatin is added to the milk or to the curd to hydrolyse at least one C₄ - C₈ fatty acid as described above.

As is known in the art, coagulation of milk can be done by the addition of acid and/or rennet. Preferably, the milk is cheese milk, which is milk that has been standardised and/or pasteurised for a specific type of cheese. Any milk can be used, as described above.

Coagulation induces formation of curd. At the same time, a watery solution of soluble milk proteins forms, which is called whey. It is preferred to add a starter culture, either to the milk or to the curd. A starter culture, as is known in the art, comprises at least one enzyme, preferably an enzyme mix, that degrades milk components such as fat and proteins to solidify the cheese, and to induce flavour formation. Addition of patatin, to the milk, the curd or the starter culture, as in the present method, enhances flavour formation and is therefore highly beneficial in the making of cheese.

Draining the curd means that at least part of the whey is separated from the curd. This is commonly done by pressing, but other methods are conceivable. Preferably, the curd is shaped and/or salted, also, in order to obtain an attractive shape and taste, and in order to prevent the growth of microorganisms.

The result of sufficient draining is formation of a fresh cheese. Certain cheeses are customarily eaten as a fresh cheese, but often, a ripening step increases the quality of he cheese. Thus, optionally the fresh cheese is allowed to ripen. Ripening the fresh cheese means that the drained curd is left standing for an amount of time that is dependent on the type of cheese. Soft ripened cheeses usually have a minimal ripening time, but other types of cheese may require much more time, such as months or years, to ripen. Ripening for sufficient time results in the final cheese.

Preferably, cheese such as Italian-type cheese, blue cheese and/or enzyme-modified cheese can be prepared following this method, and use of patatin for enhancing the flavour of Italian-type cheese, blue cheese and/or enzyme modified cheese is therefore preferred.

The invention will now be illustrated by the following non-limiting examples.

### Example 1: Feasibility of Patatin-catalyzed Lipolysis in acid-coagulated whole milk

1 L of whole milk was heated in a 2 L beaker on a laboratory hot plate. Upon boiling the heating element was switched off and acetic acid was added under stirring from 30% solution until a curd formed. Curds were collected using a cheese cloth in a colander and allowed to cool. The curds were divided into two fractions. A 10 mL 10% w:v solution of patatin (Solanic 206P) were poured slowly over one fraction, while 10 mL of demineralized water were poured over the other to serve as an untreated control. Residual whey was removed from the curds by gentle pressing. Within 5 minutes, the patatin-treated curd mass developed a strong cheese scent, while the untreated control retained a boiled milk scent.

### Example 2: Patatin dose-response relationship in rennet-coagulated cheese

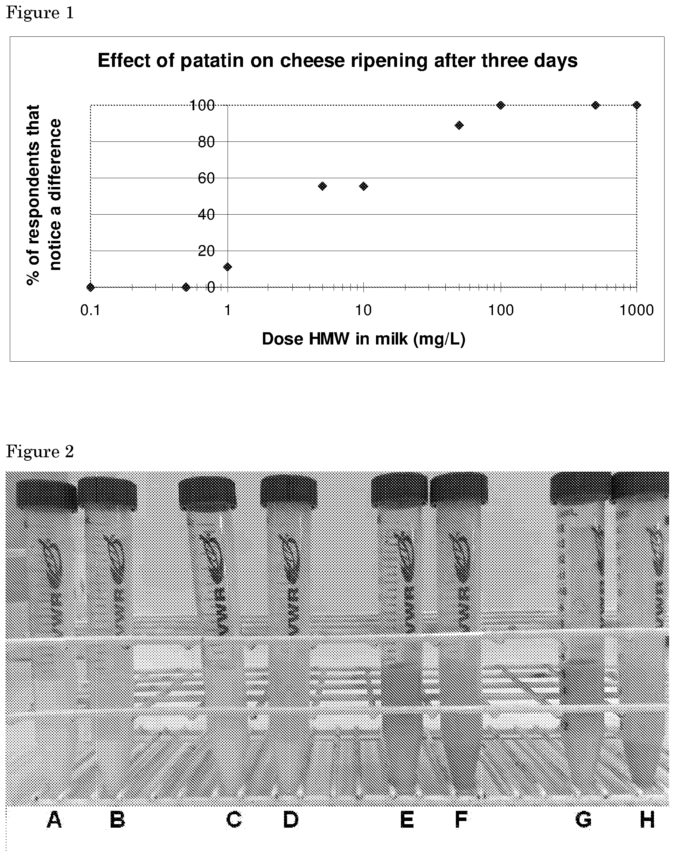

Rennet was purchased from SigmaAldrich (R5876). 500 mL aliquots of whole milk were supplemented with patatin at concentrations between 0.1 mg/L and 1.0 g/L and coagulated at 35°C for 90 minutes by the action of 10 mg/L rennet. A reference coagulated milk was prepared similarly without the addition of patatin ("HMW"). The curd fractions were recovered by straining the coagulate through cheese cloth and pressing. The resulting material was brined by complete submersion in a 90 g/L sodium chloride solution for 1 hour and allowed to ripen for three days at ambient temperature. A nine-person test panel composed of resident laboratory personnel was asked to indicate with certainty whether the scent of each cheese was noticeably different from that of the reference cheese containing no patatin. After three days, the cheeses containing 1 mg/L or more patatin had a noticeably different scent from the reference cheese (for graphic results, see Figure 1).

### Example 3: Distribution of patatin over curd and whey in rennet-induced coagulation

### Patatin Labelling

Solanic HMW potato protein isolate was used as an essentially pure patatin preparation. Coomassie Brilliant Blue was from Merck (G-250 1.15444, R-250 1.12553). PD10 gel filtration columns were from GE Healthcare. Rennet was purchased from SigmaAldrich (R5876).

A 4,0% (m:m) solution of patatin was prepared in demineralised water and incubated with equimolar amounts of either Coomassie Brilliant Blue R-250 or Coomassie Brilliant Blue G-250 and incubated at ambient temperature under constant stirring for 30 minutes. After incubation any unbound dye was removed from the protein by gel filtration on PD10 disposable gel filtration columns. Dye-labelled patatin solutions were stored at -28°C until use.

### Determination of the distribution of patatin over curd and whey via labelled patatin

5 mL aliquots of milk were supplemented with different doses of labelled patatin between 20 mg/L and 2.0 g/L. Total volume was kept constant by the addition of demi-water if required. The resulting mixtures as well as untreated milk as a control were coagulated by the addition of rennet at 10 mg/L and incubating for 90 minutes at 35°C. The resulting material was separated into curd and whey by centrifugation at 9000 g for 10 minutes. The whey was then transferred into microvials and centrifuged again at 15000 g for 10 minutes to obtain a slightly opaque solution.

Photographs of the curd and whey fractions were taken to allow the distribution of dye to be inspected optically (Figure 2). Spectrophotometric quantification of the amount of blue dye in the whey was unsuccessful because of residual turbidity that was insufficiently removed by neither centrifugation nor microfiltration.

### Determination of the distribution of patatin over curd and whey via lipase activity measurement

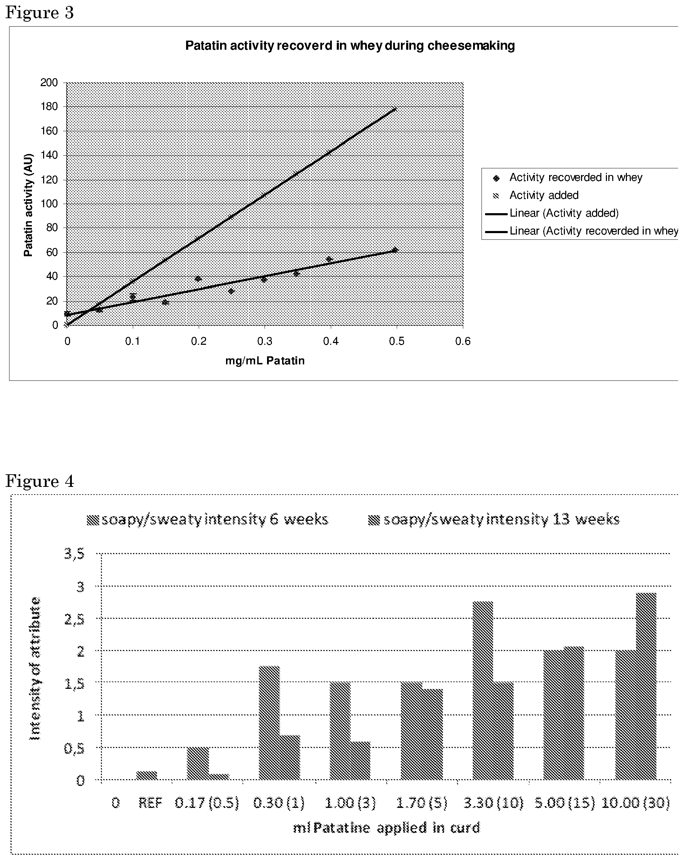

Known lipase substrates were purchased from SigmaAldrich (4-nitrophenylcaprylate, 21742). 5 mL aliquots of milk were supplemented with different doses of patatin between 50 mg/L and 500 mg/L. Total volume was kept constant by the addition of demi-water if required. The resulting mixtures as well as untreated milk as a control were coagulated by the addition of rennet at 10 mg/L and incubating for 90 minutes at 35°C. The resulting material was separated into curd and whey by centrifugation at 9000 g for 10 minutes. The whey was then transferred into microvials and centrifuged again at 15000 g for 10 minutes to obtain a slightly opaque solution. These were diluted 10000 times in demineralised water. The curd fractions were resuspended in 100 mM citrate buffer at pH 7.5 and also diluted. Aliquots of the original milk solutions were diluted to the same extent. 100 µL of these dilutions were exposed to 100 µL of 30 mM Tris-HCl pH 8.0 solution containing 2 mM of 4-nitrophenylcaprylate in a 96 well plate and analysed for absorbance at 405 nm at 10 second intervals for 5 minutes at ambient temperature using a BioRad Model 608 plate reader (Figure 3). The activity of patatin in whey is much lower than the added patatin activity, indicating that patatin predominantly fractionates in the curd upon milk coagulation.

### Example 4: Patatin inactivation

Residual lipase activity in whey may be undesired because it can lead to degradation of the remaining milk fat. This would result in the presence of volatile, odorous free fatty acids in the whey, modifying the taste with time. In addition, products prepared from the whey would contain lipolytic activity, potentially limiting their application.

Patatin (Solanic 206P HMW potato protein) was dissolved to a concentration of 1 g/L in buffer solutions of pH 5, 6 and 7, and in freshly prepared whey of pH 6.7. Kinetic degradation models of the lipase activity in these solutions were constructed by measuring residual activity upon thermal exposure in a stopped-flow system.

Whey was prepared from whole milk exposed to 10 mg/L rennet (SigmaAldrich R5876) at 30°C for 90 minutes and removing the curd by filtration through cheese cloth. Patatin solutions were exposed to temperatures between 50° and 80°C at exposure times ranging between 4 ms up to 10 s. Lipase activity was determined by measuring the increase in absorbance at 340 nm of the patatin solution acting on 4-methylumbeliferyl acetate (Alfa Aesar A12147) in 30 mM phosphate buffer of pH 8.0 for 3 minutes. The data were fitted according to Arrhenius kinetics essentially according to the method of Anton and Barret (Anton, G.E. and Barrot D.M.,2002 JAFC , 50, p.4119-25 "Inactivation of Quality-Related Enzymes in Carrots and Potatoes").

**Table 1: Thermal inactivation of patatin under different conditions. D-values represent the time required to cause a 90% reduction in activity and are reported in seconds. k is the reaction rate in reciprocal seconds, Ea is the activation energy of the inactivation reaction in kJ / mol.**

| **Condition** | **Reference T [°C]** | **k [s⁻¹]** | **Ea [kJ/mol]** | **D-value at 70 °C [s]** | **D-value at 75 °C [s]** |
|---|---|---|---|---|---|
| 30 mM phosphate Buffer pH 5.0 | 70 | 7.9 | 257.9 | 17 s | 5 s |
| 30 mM phosphate Buffer pH 6.0 | 70 | 9.7 | 268.1 | 14 s | 4 s |
| 30 mM phosphate Buffer pH 7.0 | 70 | 3.1 | 247.7 | 5.8 s | 1.7 s |
| Whey pH 6.7 | 70 | 0.28 | 204.6 | 8.2 s | 2.9 s |

During cheesemaking, whey typically undergoes a pasteurization step at 75°C. At this temperature, the data show that residual lipase activity is inactivated in a matter of seconds, specifically within at most 10 seconds.

### Example 5: Development of cheese flavour compounds by potato patatin in model cheeses

One vat of curd (200 L) was prepared as in usual cheese-making, using thermised bactofugated standardised and pasteurised cheese milk. The processing protocol for Gouda-type cheese was followed. Rennet (Kalase, CSK Food Enrichment, Leeuwarden, The Netherlands) and starter (Bos mesophilic starter, CSK Food Enrichment, Leeuwarden, The Netherlands) were added to the vat. After washing the curd, it was divided into 10 portions with different doses of patatin (Solanic 206P) mixed thoroughly. These doses ranged between 30 and 0.1 mg patatin / L cheese milk. The curds were pre-pressed, divided into 4 equal parts and each placed in a cheese vat. The resulting cheeses ( ∼350 g each) were pressed, brined, vacuum-packed and ripened at 13°C for 6 or 13 weeks.

Relevant volatile flavour components were determined and quantified by solid-phase dynamic extraction headspace GC-MS, operating in single-ion recording mode. Levels of individual fatty acids were determined in duplicate using GC-based methods according to de Jong and Badings, 1990, J High Resolution Chrom. 13:94-98. Sensory analysis was carried out by a trained expert panel (n=12) that was trained and selected via an ISO 8586 procedure. The testers represent the 10% best skilled individuals in smelling and tasting of the normal population and are regularly trained on dairy products and the Common Flavour Language (CFL). Cheeses were tested blind and in random order.

Sensory tests revealed changes in several flavour attributes associated with the addition of patatin to cheese, namely Soapy/Sweaty, Scorched, Metallic, Sweet, Diacetyl/Creamy. However, only in the case of soapy/sweaty there was a noticeable relationship with patatin dose (Figure 4). The flavour with increasing patatin dose was increasingly regarded as positively sweet and not particularly soapy.

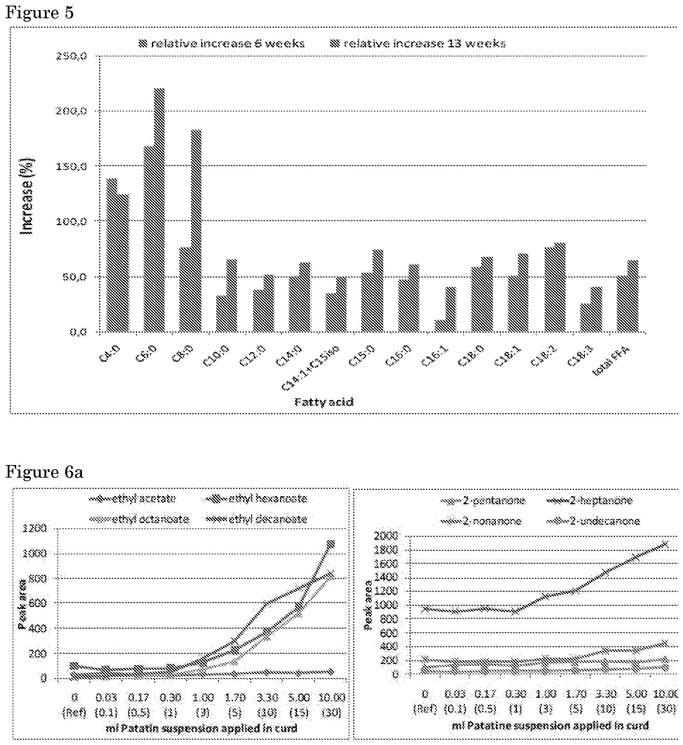

Instrumental analysis revealed increasing amounts of C₄ - C₈ fatty acids with patatin dose. The highest dose (∼30 mg/L milk) corresponded to an increase in 50% after 6 weeks and 65% after 13 weeks of ripening, relative to the control. Patatin prefers to hydrolyse C₄, C₆ and C₈ fatty acids (figure 5), which show a higher increase than other free fatty acids.

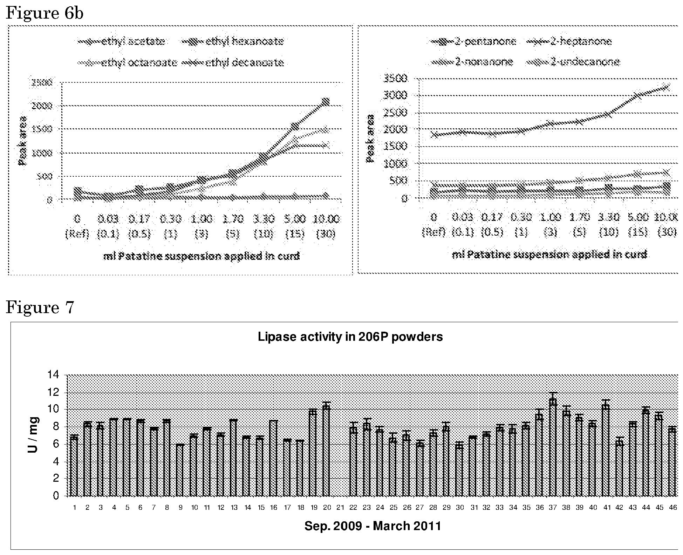

Fatty acid-derived volatiles such as ketones, aldehydes and esters increased in a dose-dependant manner with the amount of patatin added (figure 6).

### Example 6: Potato lipase shelf life

HMW potato protein powder samples covering two years of production time (Solanic 206P; patatin) were dissolved at 2% concentration in 100 mM pH 8.0 phosphate buffer. Solids were removed by centrifugation and the supernatants were analysed for lipase activity on 4 mM of paranitrophenyl caprylate (SigmaAldrich 21742) in the presence of 3 mg/mL sodium dodecyl sulphate by spectrometric measurement of the absorbance at 405 nm at 30°C. Activities were then calculated using a molar extinction coefficient of 16888 and expressed as units of lipase activity / mg powder. All analyses were performed in a single experimental series. The results show that the variation in activity is limited, despite large differences in potato cultivars, soil conditions, weather and tuber age and storage conditions. Moreover, older samples do not display lower activity than newer samples, indicating that the lipase activity is stable over at least two years and probably much longer.

### Example 7: Substrate specificity of patatin

The scientific literature contains varies studies that describe substrate specificity for patatin. These generally agree that medium-chain length paranitrophenyl-esters and saturated phospholipids, which contain between 8 and 12 carbon atoms, are the preferred substrate.

These studies agree also that patatin has no activity towards triglycerides. Surprisingly therefore, Solanics patatin products show low but clearly present activity towards short- and medium chain triglycerides having C₄ - C₈ fatty acids, and in some cases also for C₁₀ fatty acids. An activity above 0.025 mmol / min / g is considered significant for food production processes, among which cheese-making.

**Table 2: Patatin activity towards various substrates in mmol/minute/g patatin αt ambient temperature. Bold numbers indicate hydrolyses according to the present invention. Nd = not determined**

| **carbon chain length** | **triglycerides** |
|---|---|
| 2 | **0.024** |
| 4 | **0.213** |
| 6 | **0.315** |
| 8 | **0.0461** |
| 10 | **nd** |
| 12 | **0.0057** |

## Claims

1. A method for hydrolysing a fatty acid from a triglyceride that contains at least one C4 - C8 fatty acid in an emulsion or colloidal dispersion comprising milk fat, comprising subjecting the triglyceride to patatin derived from potato (*Solanum tuberosum)* in the presence of water, wherein the patatin hydrolyses a C4 - C8 fatty acid.

2. A method according to claim 1, wherein the patatin is in native form.

3. A method according to claim 1 or 2, wherein the emulsion or colloidal dispersion comprises milk.

4. A method according to claim 1 or 2, wherein the emulsion or colloidal dispersion is curd.

5. A method according to claim 4 wherein the curd is used in cheesemaking.

6. A method according to any of claims 1-5 wherein the patatin is subsequently deactivated by heating to a temperature between 50 and 80 °C.

7. A method according to any of claims 1-6 wherein the triglyceride has a water-octanol partitioning coefficient of equal to or lower than 9.2, or preferably lower than 6.3.

8. A method according to claims 3 or 4, wherein the hydrolysis results in an enhancement of the flavour of a food product.

9. A method according to claim 8 wherein the food product is cheese.

10. A method according to claim 9 wherein the cheese is Italian-type cheese, blue cheese or enzyme-modified cheese.

11. A method of making cheese, comprising the steps of
• coagulating milk to obtain a curd,
• draining the curd, and
• forming a fresh cheese, optionally followed by a ripening step,
wherein patatin derived from potato (*Solanum tuberosum*) is added to the milk or to the curd to hydrolyse a C4 - C8 fatty acid according to any of claims 1-10.

## Patentansprüche

1. Verfahren zum Hydrolysieren einer Fettsäure aus einem Triglycerid, das wenigstens eine C4 - C8-Fettsäure in einer Emulsion oder kolloidalen Dispersion, umfassend Milchfett, enthält, umfassend Aussetzen des Triglycerids dem Patatin, abgeleitet von Kartoffel (*Solanum tuberosum*) in der Gegenwart von Wasser, wobei das Patatin eine C4 - C8-Fettsäure hydrolysiert.

2. Verfahren nach Anspruch 1, wobei das Patatin in nativer Form ist.

3. Verfahren nach Anspruch 1 oder 2, wobei die Emulsion oder kolloidale Dispersion Milch umfasst.

4. Verfahren nach Anspruch 1 oder 2, wobei die Emulsion oder kolloidale Dispersion Käsebruch ist.

5. Verfahren nach Anspruch 4, wobei der Käsebruch bei der Käseherstellung verwendet wird.

6. Verfahren nach einem der Ansprüche 1 - 5, wobei das Patatin anschließend durch Erhitzen auf eine Temperatur zwischen 50 und 80 ° C deaktiviert wird.

7. Verfahren nach einem der Ansprüche 1 - 6, wobei das Triglycerid einen Wasser-Octanol-Verteilungskoeffizienten von gleich wie oder weniger als 9,2, oder vorzugsweise weniger als 6,3 hat.

8. Verfahren nach Anspruch 3 oder 4, wobei die Hydrolyse zu einer Geschmacksverstärkung eines Lebensmittelprodukts führt.

9. Verfahren nach Anspruch 8, wobei das Lebensmittelprodukt Käse ist.

10. Verfahren nach Anspruch 9, wobei der Käse, Käse italienischer Art, Blauschimmelkäse oder enzymmodifizierter Käse ist.

11. Verfahren zur Herstellung von Käse, umfassend die Schritte von
• Koagulieren von Milch, um einen Käsebruch zu erhalten,
• Abtropfenlassen des Käsebruchs und
• Bilden eines Frischkäses, optional gefolgt von einem Reifungsschritt, wobei Patatin, abgeleitet von Kartoffel *(Solanum tuberosum),* der Milch oder dem Käsebruch zugesetzt wird, um eine C4 - C8-Fettsäure nach einem der Ansprüche 1 - 10 zu hydrolysieren.

## Revendications

1. Méthode d'hydrolyse d'un acide gras à partir d'un triglycéride qui contient au moins un acide gras en C4-C8 dans une émulsion ou une dispersion colloïdale comprenant de la matière grasse de lait, comprenant la soumission du triglycéride à une patatine dérivée de pomme de terre (*Solanum tuberosum*) en présence d'eau, dans laquelle la patatine hydrolyse un acide gras en C4-C8.

2. Méthode selon la revendication 1, dans laquelle la patatine est sous forme native.

3. Méthode selon la revendication 1 ou 2, dans laquelle l'émulsion ou la dispersion colloïdale comprend du lait.

4. Méthode selon la revendication 1 ou 2, dans laquelle l'émulsion ou la dispersion colloïdale est un caillé.

5. Méthode selon la revendication 4 dans laquelle le caillé est utilisé dans la fabrication du fromage.

6. Méthode selon l'une quelconque des revendications 1 à 5, dans laquelle la patatine est ensuite désactivée par chauffage à une température entre 50 et 80 °C.

7. Méthode selon l'une quelconque des revendications 1 à 6, dans laquelle le triglycéride a un coefficient de partage eau-octanol inférieur ou égal à 9,2, ou de préférence inférieur à 6,3.

8. Méthode selon les revendications 3 ou 4, dans laquelle l'hydrolyse entraîne une amélioration de l'arôme d'un produit alimentaire.

9. Méthode selon la revendication 8, dans laquelle le produit alimentaire est un fromage.

10. Méthode selon la revendication 9, dans laquelle le fromage est un fromage de type italien, un fromage bleu ou un fromage modifié par une enzyme.

11. Méthode de fabrication de fromage, comprenant les étapes de
• coagulation de lait pour obtenir un caillé,
• égouttage du caillé, et
• formation d'un fromage frais, facultativement suivie d'une étape d'affinage,
dans laquelle de la patatine dérivée de pomme de terre *(Solanum tuberosum)* est ajoutée au lait ou au caillé pour hydrolyser un acide gras en C4-C8 selon l'une quelconque des revendications 1 à 10.
